# EUROPEAN PATENT SPECIFICATION

(11) **EP 3 402 527 B1**
(45) Date of publication and mention of the grant of the patent: **28.09.2022**
(21) Application number: 17738859.2
(22) Date of filing: 11.01.2017
(51) Int. Cl.: A61K 47/50, A61K 9/51, A61K 31/549, A61K 31/475, A61K 31/704, A61P 35/00

(54) **THERAPEUTIC NANOPARTICLES FOR THE TREATMENT OF NEUROBLASTOMA AND OTHER CANCERS**
THERAPEUTISCHE NANOPARTIKEL ZUR BEHANDLUNG VON NEUROBLASTOM UND ANDEREN KREBSARTEN
NANOPARTICULES THÉRAPEUTIQUES POUR LE TRAITEMENT DU NEUROBLASTOME ET D'AUTRES CANCERS

(30) Priority: 11.01.2016 US 201662277243 P
(43) Date of publication of application: 21.11.2018
(73) Proprietor: CorMedix Inc., Berkeley Heights, NJ 07922 (US)
(72) Inventor: DILUCCIO, Robert, Haymarket VA 20169 (US)
(74) Representative: Cullinane, Marietta Bettina
(86) International application number: PCT/US2017/013018
(87) International publication number: WO 2017/123635

(56) References cited:
- EP-A2- 1 066 830
- WO-A1-91/13628
- WO-A1-03/051902
- US-A1- 2003 044 911
- US-A1- 2008 177 217
- US-A1- 2014 140 931
- Anonymous: "Cormedix Inc. announces agreement with nanoproteagen for its proprietary nanoparticle technology, Nanopro TM, in combination with CRMD-005 for pediatric neuroblastoma", Cormedix , 11 May 2016 (2016-05-11), XP002792142, Bedminster, NJ Retrieved from the Internet: URL:http://www.cormedix.com/cormedix-inc-a nnounces-agreement-with-nanoproteagen-for- its-proprietary-nanoparticle-technology-na nopro-in-combination-with-crmd-005-for-ped iatric-neuroblastoma-2/ [retrieved on 2019-06-17]
- WONG H L ET AL: "Simultaneous delivery of doxorubicin and GG918 (Elacridar) by new Polymer-Lipid Hybrid Nanoparticles (PLN) for enhanced treatment of multidrug-resistant breast cancer", JOURNAL OF CONTROLLED RELEASE, vol. 116, no. 3, 1 December 2006 (2006-12-01), pages 275-284, XP024957602, ELSEVIER, AMSTERDAM, NL ISSN: 0168-3659, DOI: 10.1016/J.JCONREL.2006.09.007 [retrieved on 2006-12-01]
- PATIL Y ET AL: "Nanoparticle-mediated simultaneous and targeted delivery of paclitaxel and tariquidar overcomes tumor drug resistance", JOURNAL OF CONTROLLED RELEASE, vol. 136, no. 1, 21 May 2009 (2009-05-21), pages 21-29, XP026103524, ELSEVIER, AMSTERDAM, NL ISSN: 0168-3659, DOI: 10.1016/J.JCONREL.2009.01.021 [retrieved on 2009-02-05]
- DAIGELER, A ET AL.: 'Synergistic apoptotic effects of taurolidine and TRAIL on squamous carcinoma cells of the esophagus' INTERNATIONAL JOURNAL OF ONCOLOGY vol. 32, 2008, XP055399454

## Description

### Field Of The Invention

This invention relates to therapeutic compositions in general, and more particularly to therapeutic compositions for the treatment of neuroblastoma and other cancers.

### Background Of The Invention

Neuroblastoma (NB) is the most common extracranial solid cancer in childhood and the most common cancer in infancy, with an incidence of about six hundred fifty cases per year in the U.S., and a hundred cases per year in the UK. Nearly half of all neuroblastoma cases occur in children younger than two years old. Neuroblastoma causes a neuroendocrine tumor, arising from any neural crest element of the sympathetic nervous system (SNS). The neuroendocrine tumor most frequently originates in one of the adrenal glands, but it can also develop in nerve tissues in the neck, chest, abdomen, or pelvis.

Neuroblastoma is one of the few human malignancies known to demonstrate spontaneous regression from an undifferentiated state to a completely benign cellular appearance. Neuroblastoma is a disease exhibiting extreme heterogeneity, and is stratified into three risk categories: low, intermediate, and high risk. Low risk neuroblastoma disease is most common in infants and "good outcomes" are common with observation only or surgery, whereas high risk neuroblastoma disease is difficult to treat successfully even with the most intensive multi-modal oncological therapies available.

Esthesioneuroblastoma, also known as olfactory neuroblastoma, is believed to arise from the olfactory epithelium and its classification remains controversial. However, since it is not a sympathetic nervous system malignancy, esthesioneuroblastoma is a distinct clinical entity and is not to be confused with neuroblastoma.

### Signs And Symptoms

The first symptoms of neuroblastoma are often vague, making diagnosis difficult. Fatigue, loss of appetite, fever and joint pain are common. Symptoms depend on primary tumor locations and metastases if present.
- In the abdomen, a tumor may cause a swollen belly and constipation.
- A tumor in the chest may cause breathing problems.
- A tumor pressing on the spinal cord may cause weakness and an inability to stand, crawl, or walk.
- Bone lesions in the legs and hips may cause pain and limping.
- A tumor in the bones around the eyes or orbits may cause distinct bruising and swelling.
- Infiltration of the bone marrow may cause pallor from anemia.

Neuroblastoma often spreads to other parts of the body before any symptoms are apparent, and 50-60% of all neuroblastoma cases present with metastases.

The most common location for neuroblastoma to originate (i.e., the location of the primary tumor) is on the adrenal glands. This occurs in 40% of localized tumors and in 60% of cases of widespread neuroblastoma disease. Neuroblastoma can also develop anywhere along the sympathetic nervous system chain from the neck to the pelvis. Frequencies in different locations include: neck (1%), chest (19%), abdomen (30% non-adrenal), or pelvis (1%). In rare cases, no primary tumor can be discerned.

Rare but characteristic presentations include transverse myelopathy (tumor spinal cord compression, 5% of cases), treatment-resistant diarrhea (tumor vasoactive intestinal peptide secretion, 4% of cases), Homer's syndrome (cervical tumor, 2.4% of cases), opsocinus myoclonus syndrome and ataxia (suspected paraneoplastic cause, 1.3% of cases), and hypertension (catecholamine secretion or renal artery compression, 1.3% of cases).

### Cause

The etiology of neuroblastoma is not well understood. The great majority of cases are sporadic and non-familial. About 1-2% of cases run in families and have been linked to specific gene mutations. Familial neuroblastoma in some cases is caused by rare germline mutations in the anaplastic lymphoma kinase (ALK) gene. Germline mutations in the PHOX2A or KIF1B gene have been implicated in familial neuroblastoma as well. Neuroblastoma is also a feature of neurofibromatosis type 1 (NF1), also called von Recklinghausen's disease, and the Beckwith-Wiedemann syndrome.

MYCN oncogene amplification within the tumor is a common finding in neuroblastoma. The degree of amplification shows a bimodal distribution: either 3- to 10-fold, or 100- to 300-fold. The presence of this mutation is highly correlated to advanced stages of disease (Ref. 1).

Duplicated segments of the LMO1 gene within neuroblastoma tumor cells have been shown to increase the risk of developing an aggressive form of the cancer (Ref. 2).

Neuroblastoma has been linked to copy-number variation within the NBPF10 gene, which results in the lq21.1 deletion syndrome or 1q21.1 duplicate syndrome (Ref. 3).

Several risk factors for neuroblastoma have been proposed and are the subject of ongoing research. Due to the characteristic early onset of neuroblastoma, many studies have focused on parental factors relating to conception and gestation. Factors investigated have included occupation (i.e., exposure to chemicals in specific industries), smoking, alcohol consumption, use of medicinal drugs during pregnancy and birth factors, however, results have been inconclusive (Ref. 4).

Other studies have examined possible links with atopy and exposure to infection early in life (Ref. 5), the use of hormones and fertility drugs (Ref. 6), and maternal use of hair dye (Ref. 7).

### Biochemistry

In about 90% of neuroblastoma cases, elevated levels of catecholamines or their metabolites are found in the urine or blood. Catecholamines and their metabolites include dopamine, homovanillic acid (HVA) and/or vanillymandelic acid (VMA)(Ref. 8).

### Treatment

When a neuroblastoma lesion is localized, it is generally curable. However, long-term survival for children with advanced disease older than 18 months of age is poor despite aggressive multimodal oncological therapy, e.g., intensive chemotherapy, surgery, radiation therapy, stem cell transplant, use of the differentiation agent isotrentinoin (also called 13-*cis*-retinoic acid), immunotherapy with anti-GD2, immunotherapy with anti-GD2 monoclonal antibody therapy, etc.

Biologic and genetic characteristics have been identified which, when added to classic clinical staging, has allowed patient assignment to risk groups for planning the intensity of treatment. These criteria include the age of the patient, the extent of disease spread, microscopic appearance, and genetic features including DNA ploidy and N-myc oncogene amplification (N-myc regulate micro RNAs), and are used to categorize patients into low, intermediate, and high risk disease states. A recent biology study (COG ANBL00B1) analyzed 2687 neuroblastoma patients and the spectrum of risk assignment was determined: 37% of neuroblastoma cases are low risk, 18% are intermediate risk, and 45% are high risk. There is some evidence that the high and low risk types are caused by different mechanisms, and are not merely two different degrees of expression of the same mechanism (Ref. 9).

The therapies for these different risk categories are very different.
- Low risk disease can frequently be observed without any treatment at all, or cured with surgery alone.
- Intermediate risk disease is treated with surgery and chemotherapy.
- High risk neuroblastoma is treated with intensive chemotherapy, surgery, radiation therapy, bone marrow/hematopoietic stem cell transplantation, biological-based therapy with 13-*cis*-retinoic acid (isotretinoin or Accutane) and antibody therapy usually administered with the cytokines GM-CSF and IL-2 cytokines.

With current treatments, patients with low and intermediate risk neuroblastoma disease have an excellent prognosis, with cure rates above 90% for low risk and 70-90% for intermediate risk. In contrast, over the past two decades, therapy for high risk neuroblastoma has yielded a cure rate of only about 30%. The addition of antibody therapy has raised survival rates for high risk neuroblastoma disease significantly. In March 2009, an early analysis of a Children's Oncology Group (COG) study with 226 high risk patients showed that two years after stem cell transplant, 66% of the group randomized to receive the ch14.18 antibody (with GM-CSF and IL-2) were alive and disease-free, compared to only 46% in the group that did not receive the antibody. The randomization was stopped so all patients enrolling in the trial would receive the antibody therapy (Ref. 10).

Chemotherapy agents used in combination have been found to be effective against neuroblastoma. Agents commonly used in induction and for stem cell transplant conditioning are platinum compounds (cisplatin, carboplatin), alkylating agents (cyclophosphamide, ifosfamide, melphalan, topoisomerase II inhibitor) and vinca alkaloids (vincristine). Some newer regimens include topoisomerase I inhibitors (topotecan and irinotecan) in induction which have been found to be effective against recurrent disease.

Recent focus has been to reduce therapy for low and intermediate risk neuroblastoma patients while maintaining survival rates at 90%. A study of 467 intermediate risk patients enrolled in Clinical Trial A3961 with the Children's Oncology Group part of NIH study NCT00499616 from 1997 to 2005 confirmed the hypothesis that therapy could be successfully reduced for this risk group. Those with favorable characteristics (tumor grade and response) received four cycles of chemotherapy, and those with unfavorable characteristics received eight cycles, with three year event-free survival and overall survival stable at 90% for the entire group. Future plans are to intensify treatment for those patients with aberration of 1p36 or 11q23 chromosomes as well as for those who lack early response to treatment (Refs. 11, 12).

By contrast, the focus for the past 20 years or more has been to intensify treatment for high risk neuroblastoma patients. Chemotherapy induction variations, timing of surgery, stem cell transplant regimens, various delivery schemes for radiation, and the use of monoclonal antibodies and retinoids to treat minimal residual disease continue to be examined. Recent phase III clinical trials with randomization have been carried out to improve survival of high-risk disease:
- 1982-1985: European Neuroblastoma Study Group (ENSG1) enrolled 167 children and randomized to melphalan autologous bone marrow transplant or no further therapy (no radiation therapy given to any child). Transplant and no-transplant groups each had 65 patients, and a recent long-term follow-up report revealed significantly better 5 year event-free survival for stage 4 neuroblastoma, in over 1 year olds, in the melphalan-transplant group versus no further treatment: 33% versus 17%, respectively (Ref. 13).
- 1990-1999: European study (EU-20592 or CCLGNB-1990-11) randomized 262 high-risk children over 1 year old and revealed a higher survival rate for rapid sequence induction (10-day cycle) versus standard induction (21-day cycle) with the same total dose. Ten year event-free survival was 27% and 18%, respectively, with a non-aggressive surgical approach, no radiotherapy, and melphalan-only autologous bone marrow or stem cell transplant for both groups (Ref. 14).
- 1991-1996: Phase III trial with two sequential randomizations for 379 high risk neuroblastoma patients was carried out by the Children's Cancer Group (CCG-3891) which demonstrated improved survival with myeloablative therapy (with total body irradiation) and 13-cis-retinoic acid (Accutane) with 50 patients in each of the four groups of the study (Ref. 15).
- 1996-2003: The German (GPOH) study NB97 compared outcomes of 295 high risk neuroblastoma patients randomized for stem cell transplant or consolidation chemotherapy. Results showed increased survival with stem cell transplant (Ref. 16).
- 2000-2006: a recent study (COG-A3973) questioned the need for purged stem cells for CEM-LI (carboplatin, etoposide, melphalan, with local irradiation) transplant, and accrued 486 patients in the study. Purging stem cells was not found to improve survival rates (Ref. 17).
- 2000-2012: A concurrent study (COG-ANBL0032) determined in early review that the antibody ch14.18 with interleukin 2 and GMCSF (studied retrospectively in German GPOH NB90 and NB 97 at a lower dose and without cytokines) improved the survival rate, and with a total of 423 patients. A follow-on Phase III study COG-ANBL0931 opened January 2010 to accrue 105 patients to gather further safety and efficacy data for FDA approval (Ref. 18).
- 2002-2008: SIOP (International Society of Paediatric Oncology) formed the European SIOP Neuroblastoma Group (SIOPEN) in 1994 and activated a phase III high risk neuroblastoma protocol in 2002 (SIOP-EUROPE-HR-NBL-1) using "rapid" COJEC (8 cycles of chemotherapy given at 10 day intervals) followed by transplant randomization to CEM (carboplatin, etoposide, melphalan) or BuMel (busulfan, melphalan) and the study was amended to randomize children to ch14.18 antibody treatment with or without subcutaneous IL2 (without GM-CSF as given in the COG). This study reported the benefit of growth factors (GCSF), and all patients received retinoic acid. This trial involved 1000 patients (175 per year) (Ref. 18).
- 2005-2010: The German NB2004 randomization included MIBG therapy and topotecan use in up-front therapy and involved a total of 642 patients for all risk groups (roughly half were high risk). After transplant, the high risk protocol involved six months of cis-retinoic acid, a three month break, and another three months of retinoic acid (Ref. 19).
- 2007: The COG phase III ANBL0532 trial opened December 2007 for accrual of 495 patients and compared to single versus tandem transplants, and induction began with two cycles of topotecan (Ref. 20).

In addition to these phase III studies, some research institutions offer pilot treatment protocols. For example, St. Jude's finished (2007) testing a new up-front chemotherapy regimen in 23 children which included irinotecan and gefitnib with 16 months of maintenance chemotherapy after stem cell transplant with alternating oral 13-cis-retinoic acid and topotecan. Memorial Sloan-Kettering Cancer Center in New York offers a treatment that includes a mouse-derived monoclonal antibody, 3F8, used in protocols since the mid-1980s. This antibody is used for treating minimal residual disease or consolidation instead of stem cell transplant. A new pilot protocol COG-ANBL09P1 available for newly diagnosed (high risk) children at several Children's Oncology Group (COG) centers offers MIBG radiotherapy and chemotherapy for the transplant regimen (Ref. 21).

Some children (particularly in high risk cases) do not respond completely to frontline treatment (with a complete response or very good partial response) and are labeled refractory. These "refractory" children are removed from the frontline therapy (clinical trial) and are eligible for clinical trials using new therapies. Many high risk children have a good response to frontline therapy and achieve a remission, but later the disease recurs (relapse). These children are also eligible for new therapies being tested in clinical trials.

Chemotherapy with topotecan and cyclophosphamide is frequently used in refractory settings and after relapse. A randomized study (2004) with 119 patients (comparing topotecan alone to topotecan and cyclophosphamide) revealed a 31% complete or partial response rate with two year progression-free survival at 36% in the topotecan and cyclophosphamide group. Irinotecan (intravenous or oral) and oral temozolomide are also used in refractory and recurrent neuroblastoma (Ref. 22).

Many phase I and phase II trials are currently testing new agents against neuroblastoma in children who have relapsed or are resistant to initial therapy. Investigators are currently studying new agents, alone and in new combinations, using small molecule targeted therapy, 131-1 MIBG radiation therapy, angiogenesis agents, new monoclonal antibodies, vaccines, oncolytic viruses, as well as new myeloablative regimens.

A group of 16 children's hospitals in the United States, known as the New Advances in Neuroblastoma Therapy (NANT) consortium, coordinates the I-131 MIBG radiation therapy trials. The NANT consortium also offers trials using an oral powder formulation of fenretinide, intravenous fenretinide, bisphosphonate (Zometa) with other agents, and combining I-131 MIBG with the inhibitor vorinostat (Ref. 23).

Other research study groups such as The Neuroblastoma and Medulloblastoma Translational Research Consortium (NMTRC) also conduct clinical trials to treat relapse neuroblastoma. Institutions in Europe are studying novel therapies to treat relapse, including haploidentical stem cell transplant. Many hospitals conduct their own institutional studies as well.

The protein p53 is believed to play a role in the development of resistance to chemotherapy. A November 2009 study in mice shows that activating the tumor suppressor p53 with a new drug, nutlin-3, may slow tumor growth. In this study, physician Tom Van Maerken of Ghent University Hospital in Belgium and his colleagues used nutlin-3 to neutralize MDM2, a protein that binds to the p53 protein and obstructs p53's ability to trigger programmed cell death. Earlier studies have shown that nutlin-3 can specifically prevent MDM2 from disabling p53.

### Objects Of The Invention

It has been observed that the drug taurolidine has the ability to enhance the activity of a number of oncologic drugs.

As a result, one object of this invention is to harness the synergistic effect of taurolidine on these oncologic drugs so as to allow for greater efficiency and reduced toxicity associated with the oncologic drugs.

Still another object of this invention is to create nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating which is configured to release the one or more oncologic drugs and taurolidine locally to the site of a cancer, e.g., a tumor. In one preferred form of the invention, the coating is configured to prevent premature exposure of the one or more oncologic drugs and taurolidine to the body prior to delivery to the site of the cancer, e.g., a tumor. This can be important in order to prevent undesirable side effects from the one or more oncologic drugs, the premature hydrolization of the taurolidine, etc. According to the invention, the coating comprises an absorbable polymer.

Yet another object of this invention is to provide nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating, wherein the coating is configured to target the nanoparticle to the site of a cancer (e.g., a tumor) so as to improve the efficacy of the oncologic drugs and taurolidine for treatment of the cancer. In one preferred form of the invention, the coating comprises binding molecules which are configured to target delivery of the nanoparticle to specific tissue.

And another object of this invention is to provide nanoparticles specifically configured for the treatment of neuroblastoma and/or other specific cancers.

### Taurolidine In General

Taurolidine (bis(1,1-dioxoperhydro-1,2,4-thiadiazinyl-4)-methane) has antimicrobial and antilipopolysaccharide properties. It is derived from the amino acid taurine. Its immunomodulatory action is reported to be mediated by priming and activation of macrophages and polymorphonuclear leukocytes.

Taurolidine has been used to treat patients with peritonitis and as an antiendoxic agent in patients with systemic inflammatory response syndrome. It is a life-saving antimicrobial for severe abdominal sepsis and peritonitis. Taurolidine is active against a wide range of micro-organisms that include gram positive bacteria, gram negative bacteria, fungi, mycobateria and also bacteria that are resistant to various antibiotics such as methicillin-resistant Staphylococcus aureus (MRSA), vancomycin intermediate staphylococcus aureus (VISA), vancomycin-resistant staphylococcus aureus (VRSA), oxacillin resistant staph aureus (ORSA) and vancomycin-resistant enterococci (VRE). Additionally, taurolidine demonstrates some anti-tumor properties, with positive results seen in early-stage clinical investigations using the drug to treat gastrointestinal malignancies and tumors of the central nervous system.

Taurolidine is the active ingredient of anti-microbial catheter lock solutions for the prevention and treatment of catheter-related blood stream infections (CRBSIs) and is suitable for use in all catheter-based vascular access devices. Bacterial resistance against taurolidine has never been observed in various studies.

Taurolidine acts by a non-selective chemical reaction. In aqueous solution, the parent molecule taurolidine forms equilibrium with taurultam and Nhydroxymethyl taurultam, with taurinamide being a downstream derivative. The active groups of taurolidine are N-methylol derivatives of taurultam and taurinamide, which react with the bacterial cell wall, cell membrane, and proteins as well as with the primary amino groups of endo- and exotoxins. Microbes are killed and the resulting toxins are inactivated; the destruction time in vitro is 30 minutes. Pro-inflammatory cytokines and enhanced tumor necrosis factor (TNF) levels are reduced when taurolidine is used as a catheter lock solution. Taurolidine decreases the adherence of bacteria and fungi to host cells by destructing the fimbriae and flagella and thus prevent biofilm formation.

A dose of 5g of taurolidine over 2 hours, every 4 hours, for at least 48 hours, has been given intravenously for the treatment of various sepsis conditions.

### The Synergistic Activity Of Taurolidine Has Been Observed In The Following Applications Involving The Use Of Oncologic Drugs

Karlisch et al. (Ref. 24) observed the effects of TNF-related apoptosis-inducing ligand (TRAIL) and taurolidine on apoptosis and proliferation in human rhabdomyosarcoma, leiomyosarcoma and epithelioid cell sarcoma. Soft tissue sarcomas (STS) are a heterogeneous group of malignant tumors representing 1% of all malignancies in adults. Therapy for STS should be individualized and multimodal, but complete surgical resection with clear margins remains the mainstay of therapy. Disseminated soft tissue sarcoma still represents a therapeutic dilemma. Commonly used chemotherapeutic agents such as doxorubicin and ifosfamide have proven to be effective in fewer than 30% in these cases. Therefore, Karlisch et al. tested the apoptotic and anti-proliferative in vitro effects of the TNF-related apoptosis-inducing ligand (TRAIL) and taurolidine on rhabdomyosarcoma (A-204), leiomyosarcoma (SK-LMS-1) and epithelioid cell sarcoma (VA-ES-BJ) cell lines. Viability, apoptosis and necrosis were quantified by FACS analysis (propidium iodide/Annexin V staining). Gene expression was analyzed by DNA microarrays and the results validated for selected genes by rtPCR. Protein level changes were documented by western blot analysis. Cell proliferation was analyzed by bromodeoxyuridine (BrdU) ELISA assay. The single substances TRAIL and taurolidine significantly induced apoptotic cell death and decreased proliferation in rhabdomyosarcoma and epithelioid cell sarcoma cells. The combined use of TRAIL and taurolidine resulted in a synergistic apoptotic effect in all three cell lines, especially in rhabdomyosarcoma cells, leaving 18% viable cells after 48 hours of incubation (p<0.05). Analysis of the differentially regulated genes revealed that taurolidine and TRAIL influence apoptotic pathways, including the TNF-receptor associated mitochondrial pathway. Microarray analysis revealed remarkable expression changes in a variety of genes which are involved in different apoptotic pathways and cross-talk to other pathways at multiple levels. This in vitro study demonstrates that TRAIL and taurolidine synergize in inducing apoptosis and inhibiting proliferation in different human STS cell lines. Effects on gene expression differ relevantly in the sarcoma entities. These results provide experimental support for in vivo trials assessing the effect of TRAIL and taurolidine in STS and sustain the approach of individualized therapy.

Harati et al. (Ref. 25) observed TRAIL and taurolidine enhance the anticancer activity of doxorubicin, trabectedin and mafosfamide in HT1080 human fibrosarcoma cells. Disseminated fibrosarcoma still represents a therapeutic dilemma due to the lack of effective cytostatics. Therefore tumor necrosis factor (TNF)-related apoptosis-inducing ligand (TRAIL) and taurolidine, in combination with established and new chemotherapeutic agents on human fibrosarcoma (HT1080), was observed to improve apoptosis.

Materials and Methods: Human fibrosarcoma cells (HT1080) were incubated with doxorubicin, mafosfamide and trabectedin, both alone and in combination with taurolidine and TRAIL. Vital, apoptotic and necrotic cells were quantified using flow cytometric analysis. Cell proliferation was analyzed using a bromodeoxyuridine (BrdU) ELISA assay.

Results: Single application of doxorubicin and trabectedin induced apoptotic cell death and significantly reduced the proliferation of HT1080 cells. In combination treatment, the addition of taurolidine and TRAIL resulted in a stronger reduction in the degree of cell viability when compared to a single treatment. Trabectedin and taurolidine displayed a greater potential for inhibiting proliferation than did doxorubicin alone.

Conclusion: When combined with TRAIL and taurolidine, treatment with doxorubicin and trabectedin demonstrated stronger apoptosis-inducing and antiproliferative effects.

Martinotti et al. (Ref. 26) studied in vitro screening of synergistic ascorbate-drug combinations for the treatment of malignant mesothelioma. Malignant mesothelioma (MMe) is a lethal tumor arising from the mesothelium of serous cavities as a result of exposure to asbestos. Current clinical studies consist of combined treatments, but an effective therapy has not been established yet and there is an urgent need for new curative approaches. Ascorbate is a nutrient that is also known as a remedy in the treatment of cancer. In this study, Martinotti et al. tested the cytotoxicity of ascorbate to MMe cells in combination with drugs used in MMe therapy, such as cisplatin, etoposide, gemcitabine, imatinib, paclitaxel, and raltitrexed, as well as with promising antitumor compounds like taurolidine, a -tocopherol succinate, and epigallocatechin-3- gallate (EGCG). Dose-response curves obtained for each compound by applying the neutral red uptake (NRU) assay to MMe cells growing in vitro allowed IC50 values to be measured for each compound used singularly. Thereafter, NRU data obtained from each ascorbate/drug combination were analyzed through Tallarida's isobolograms at the IC50 level (Tallarida, 2000), revealing synergistic interactions for ascorbate/gemcitabine and ascorbate/EGCG. These results were further confirmed through comparisons between theoretical additivity IC50 and observed IC50 from fixed-ratio dose-response curves, and over a broad range of IC levels, by using Chou and Talalay's combination index (Chou and Talalay, 1984). Synergistic interactions were also shown by examining apoptosis and necrosis rates, using the caspase 3 and lactic dehydrogenase assays, respectively. Hence, data indicate that ascorbate/gemcitabine and ascorbate/EGCG demonstrates a synergistic affect on the viability of MMe cells and suggest their possible use in the clinical treatment of this problematic cancer.

Daigeler et al. (Ref. 27) observed synergistic apoptotic effects of taurolidine and TRAIL on squamous carcinoma cells of the esophagus. The treatment of choice for esophageal cancer is considered surgical resection, but the median survival rate at 20 months after treatment is discouraging. The benefit of adjuvant or neoadjuvant radiation or chemotherapy is limited and, to date, benefits have only been identified for certain tumor stages. Therefore, new therapeutic options are required. As alternative chemotherapeutics, Daigeler et al. tested the antibiotic taurolidine on KYSE 270 human esophageal carcinoma cells alone and in combination with rhTRAIL (recombinant human TNF-related apoptosis-inducing ligand). Viability, apoptosis and necrosis were visualized by TUNEL assay and quantitated by FACS analysis. Gene expression was analyzed by RNA micro array. The most effective concentration of taurolidine as a single substance (250 m mol/l) induced apoptosis to a maximum of 40% after a 12 hour dose, leaving 4% viable cells after 48 hours; by comparison, rhTRAIL did not have a significant effect. The combination of both substances doubled the effect of taurolidine alone. Gene expression profiling revealed that taurolidine downregulated endogenous TRAIL, TNFRSF1A, TRADD, TNFRSF1B, TNFRSF21 and FADD, as well as MAP2K4, JAK2 and Bcl2, Bcl2l1, APAF1 and caspase-3. TNFRSF25, cytochrome-c, caspase-1, -8, -9, JUN, GADD45A and NFKBIA were upregulated. TRAIL reduced endogenous TRAIL, Bcl211 and caspase-1 expression. BIRC2, BIRC3, TNFAIP3, and NFKBIA were upregulated. The combined substances upregulated endogenous TRAIL, NFKBIA and JUN, whereas DFFA and TRAF3 were downregulated compared to taurolidine as single substance. Daigeler et al. concluded that taurolidine overcomes TRAIL resistance in KYSE 270 cells. Synergistic effects are dependent on the same and on distinct apoptotic pathways which, jointly triggered, result in an amplified response. Several apoptotic pathways, including the TNF-receptor associated and the mitochondrial pathway, were differentially regulated by the substances on a gene expression level. Additional transcription factors seem to be influenced, NFKB in particular. Endogenous TRAIL expression is increased by the combination of substances, whereas it is reduced by each single substance. Taking into consideration that the non-toxic taurolidine was able to reduce rhTRAIL toxicity and dose, a combined therapy with taurolidine and rhTRAIL may offer new options for treatment in esophageal cancer.

Chromik et al. (Ref. 28) observed synergistic effects of taurolidine and rhTRAIL for apoptosis induction in HCT15 colon carcinoma cells. Induction of apoptosis in tumor cells by TRAIL (TNF-related apoptosis-inducing ligand) is a promising therapeutic option in oncology, although toxicity and resistance against TRAIL are limiting factors. Taurolidine is an anti-neoplastic agent with low toxicity and thus a potential candidate for a combined therapy with TRAIL. The aim of the Chromik et al. study was to evaluate the combined treatment of TRAIL and taurolidine in the HCT15 human colonic carcinoma cell line. HCT15 cells were cultured and incubated with increasing concentrations of recombinant human TRAIL (50 to 500 ng/mL) or taurolidine (50 to 1000 m mol/1) to evaluate the dose dependent effects of both substances concerning apoptosis and necrosis. Thereafter, cells were incubated in a second experiment with TRAIL (50 and 250 ng/mL) or taurolidine (100 and 1000 m mol/l) alone as well as with combinations of both agents in different concentrations. At different time points (3 to 36 hours), cell viability, apoptosis, and necrosis were quantified by FACS analysis with propidium iodide and Annexin V staining. Results were expressed as means, statistical analysis was carried out by ANOVA, pair comparison by Tukey-test. P values < 0.05 were considered as statistically significant. Incubation with taurolidine resulted in a dose dependent cell death induction with maximum effects of 100 m mol/l and 1000 m mol/l after 24 hours and 36 hours, resulting in a reduction of viable cells from 60% to 17-33%. 250 Ng/mL and 500 ng/mL TRAIL led to a decrease of viable cells from 70% to 6-7% in as early as 6 hours, with a partial recovery of viable cells to 13% after 36 hours. Combined treatment of taurolidine (100 m mol/l) and TRAIL (50 ng/mL) caused a sustained induction of apoptosis after 24 hours and 36 hours, showing a significant synergistic effect of both substances, clearly exceeding a simply additive effect. After 24 hours incubation with taurolidine (100 m mol/l) and TRAIL (50 ng/mL), only 2.1% of the cells were viable compared to 43.9% for taurolidine 100 m mol/l and 17.7% for TRAIL 50 ng/m alone. Similar results were obtained after 36 hours. Chromik et al. showed for the first time a synergistic effect of recombinant human TRAIL and taurolidine on apoptosis induction of human colon carcinoma cells in vitro. Combined treatment with TRAIL and taurolidine resulted in sustained cell death which was superior to single agent application. Combination of TRAIL with the non-toxic taurolidine offers a novel therapeutic rationale in oncological therapy.

Braumann et al. (Ref. 29) observed the local and systemic chemotherapy with taurolidine and taurolidine/heparin in colon cancer-bearing rats undergoing laparotomy. Experimental studies in the therapy of malignant abdominal tumors have shown that different cytotoxic agents suppress the intraperitoneal (i.p.) tumor growth. Nevertheless, a general accepted approach to prevent tumor recurrences does not exist. Following subcutaneous (s.c.) and i.p. injection of 104 colon adenocarcinoma cells (DHD/K12/TRb), the influences of both taurolidine or taurolidine/heparin on i.p. and s.c. tumor growth was investigated in 105 rats undergoing midline laparotomy. The animals were randomized into 7 groups and operated on during 30 minutes. To investigate the i.p. (local) influence of either taurolidine or heparin on tumor growth, the substances were applied. I.p. systemic and i.p. effects were evaluated after i.v. injection of the substances. Both application forms were also combined to analyze synergistic effects. Tumor weights, as well as the incidence of abdominal wound metastases, were determined four weeks after the intervention. To evaluate the effects of the agents, blood was taken to determine the peripheral leukocytes counts. I.p. tumor growth in rats receiving i.p. application of taurolidine (median 7.0 mg, P = 0.05) and of taurolidine/heparin (median 0 mg, P = 0.02) was significantly reduced when compared to the control group (median 185 mg). The simultaneous instillation of both agents also reduced the i.p. tumor growth (median 4 mg, P = 0.04), while the i.v. injection of the substances caused no local effect. In contrast, the s.c. tumor growth did not differ among all groups. In all groups, abdominal wound recurrences were rare and did not differ. Independent of the agents and the application form, the operation itself caused a slight leukopenia shortly after the operation and a leukocytosis in the following course. I.p. therapy of either taurolidine or in combination with heparin inhibits local tumor growth and abdominal wound recurrences in rats undergoing midline laparotomy. Neither the i.p. nor the i.v. application or the combination of the two agents influenced the s.c. tumor growth. The substances did not alter the changes of peripheral leukocytes.

Stendel et al. (Ref. 30) observed the enhancement of Fas-ligand-mediated programmed cell death by taurolidine. Taurolidine was found to have a direct and selective antineoplastic effect on brain tumor cells. The ability of taurolidine to exert antineoplastic action by enhancement of Fas-mediated apoptosis in different malignant glioma cell lines was investigated.
Materials and Methods: human derived U373 cells were cultured and incubated with taurolidine and the median inhibitory concentration (IC50) was calculated. Flow cytometric analysis was performed to assess changes in DNA content. The cells were qualitatively and quantitatively examined using light microscopy and electron microscopy. LN-18 and LN- 229 cells were incubated in the absence or presence of either Fas-ligand, taurolidine or respective combinations thereof. The cell viability was determined by adding a double concentrated WST-1 reagent. The activity of the mitochondrial succinate reductase was measured in an ELISA reader.
Results: the exposure of 0373 cells to taurolidine led to a concentration-dependent (IC50 35.8±2.2 m g/mL) loss of cell viability. Flow cytometric analysis demonstrated a concentration dependent appearance of DNA debris in the sub-G0/G1 region. In the presence of 6.25 vol.% Fas-ligand, LN-18 cells displayed more than 90% loss of cell viability, whereas the viability of LN-229 cells was reduced only at higher concentrations of Fas-ligand. Taurolidine by itself did not appreciably affect the viability of LN-18 cells in the investigated concentration range, but was able to enhance the effect of Fas-ligand on LN-18 cells. The exposure of LN-229 cells to taurolidine alone caused an appreciable loss of cell viability by about 70% at the highest concentration tested. Cell destruction by Fas-ligand (10 vol.%) was enhanced in the presence of taurolidine.
Conclusion: the antineoplastic activity of taurolidine seems to be partially based on the enhancement of Fas-ligand-induced apoptosis. In addition, taurolidine was demonstrated to have an antineoplastic effect independent of Fas-ligand. Perhaps taurolidine exerts antineoplastic activity based on different mechanisms.

In another study, Braumann et al. (Ref. 31) assessed the influence of intraperitoneal and systemic application of taurolidine and taurolidine/heparin during laparoscopy on intraperitoneal (i.p.) and subcutaneous (s.c.) tumor growth in rats. The researchers investigated the problem and possible pathomechanisms of port-site metastases after laparoscopic resection of malignant tumors. A generally accepted approach to prevent these tumor implantations does not exist so far. After s.c. and i.p. injection of 104 cells of colon adenocarcinoma (DHD/K 12/TRb), the influences of either taurolidine or taurolidine/heparin on i.p. and s.c. tumor growth were investigated in 105 rats undergoing laparoscopy with carbon dioxide. The animals were then randomized into seven groups. A pneumoperitoneum was established using carbon dioxide for 30 minutes (8 mmHg). Three incisions were used: median for the insufflation needle, and a right and left approach in the lower abdomen for trocars. To investigate the i.p. (local) influence of taurolidine and heparin on tumor growth, the substances were instilled i.p. Systemic effects were expected when the substances were applied i.v. Synergistic influences were tested when both application forms were combined. The number and the weight of tumors, as well as the incidence of abdominal wall and port-site metastases, were determined four weeks after intervention. Blood was taken to evaluate the influences of taurolidine and heparin on systemic immunological reactions: seven days before laparoscopy, two hours, two days, seven days, and four weeks after operation, and the peripheral lymphocytes were determined. I.p. tumor weight in rats receiving taurolidine (median 7 mg) and taurolidine/heparin (0 mg) i.p. was significantly reduced when compared to the control group (52 mg) (P = 0.001). There was no difference of s.c. tumor growth among the groups (P = 0.4). Trocar recurrences were decreased when taurolidine was applied i.p. (3/15), i.p.i.v. (4/15), and i.p. in combination with heparin (4/15) in comparison to the control group (10/15). Immediately after intervention, treated and untreated groups showed a peripheral lymphopenia. The i.p. therapy with taurolidine and the combination with heparin inhibits the i.p. tumor growth and trocar recurrences. Neither the i.p. nor the systemic application, or the combination of taurolidine and heparin reduced the s.c. tumor growth. The intervention caused a lymphopenia which was compensated on day two.

Monson et al. (Ref. 32) observed taurolidine inhibits tumor necrosis factor (TNF) toxicity, with evidence of TNF and endotoxin synergy. The use of recombinant tumor necrosis factor (TNF) in the treatment of solid tumors has been limited by life threatening toxicity. In addition, TNF may be a major mediator of the effect of endotoxins. Recent evidence suggests that a synergism between endotoxin (at the picogram level) and TNF may contribute to this toxicity. The use of the anti-endotoxin taurolidine may reduce TNF toxicity by interfering with this synergy. C57/BL6 mice (n = 140) received toxic doses (12 micrograms/mouse IV) of TNF. Four groups were studied. Group A received taurolidine (200 mg/kg IV) 30 minutes before TNF, group B received TNF followed 30 minutes later by taurolidine (200 mg/kg IV), group C received an identical volume (0.5 ml) of normal saline 30 minutes prior to TNF, and group D received taurolidine (200 mg/kg IP) 45 minutes before TNF. The mortality rate of those mice receiving intravenous taurolidine 30 minutes prior to TNF was 8.8%. This was significantly less (P < 0.005) than the mortality rate achieved in groups B, C and D (33% vs 39.4% vs 50%). Further experiments employing an MTT (3-(4,5-dimethylthinzol-2-microliters)-2,5-diphenyl tetrazolinm bromide) assay showed that this was not due to direct interaction of taurolidine with TNF but is likely to be due to interference with the synergistic effects of endotoxin and TNF. It was also demonstrated in cotherapy studies in a murine model that taurolidine did not reduce the anti-tumour efficacy of TNF against the TNF sensitive mouse fibrosarcoma cell line Meth-A sarcoma.

### Synergistic Activity Of Taurolidine With Drugs For Treatment Of Neuroblastoma

Eschenburg et al. (Ref. 33) observed that taurolidine cooperates with antineoplastic drugs in neuroblastoma cells. In neuroblastoma, the outcome of stage 4 disease remains poor and the development of novel therapeutic approaches is thus urgently needed. Taurolidine, which is known to inhibit catheter infections, has exhibited antineoplastic activity in various cancers. The growth of neuroblastoma cell lines is inhibited by taurolidine as recently demonstrated. Further analysis disclosed a significant negative growth effect of taurolidine on the four neuroblastoma cell lines SH-EP TET21N, SK-N-AS, SK-N-BE(2)-M17 and SK-N-SH. Detected IC50 (51-274 mM; 48 hours) are promising and correspond to clinically-achievable plasma levels. Apoptosis was induced (76-86%; 48 hours) in a time-dependent manner mediated by a simultaneous activation of the intrinsic and extrinsic pathways. This was confirmed by cleavage of caspases -3, -8 and -9 and abrogation of apoptosis by pan-caspase inhibition. Application of taurolidine resulted in a significant enhancement of cytotoxic drugs vincristine/doxorubicin (2/3 of 4 cell lines) making taurolidine a promising candidate to be included in neuroblastoma therapy regimens in the future.

Eschenburg et al. (Ref. 33) also observed taurolidine specifically inhibits the growth of neuroblastoma cell lines in vitro. The anti-neoplastic properties of taurolidine have been demonstrated on a variety of human cancer cells. However, data on neuroblastoma is lacking. Therefore, Eschenburg et al. sought to evaluate the effect of taurolidine on growth of neuroblastoma cell lines.
Materials and methods: Neuroblastoma SK-N-BE(2)-M17 and SK-NSH cells and nonmalignant human umbilical vein endothelial cells (as controls) were incubated with increasing concentrations of taurolidine (100, 250, 500 mM). Cell growth was examined after 12, 24, and 48 hours of exposure.
Results: inhibition of cell growth by taurolidine was seen in both malignant cell lines. When compared with human umbilical vein endothelial cells, the neuroblastoma cell lines were significantly more responsive to taurolidine. Conclusions: the observed negative impact on cell growth, highly distinctive in SK-N-BE(2)-M17 and SK-N-SH, implies a taurolidine-specific mode of action that appears dependent on differences on cellular and molecular levels. Further investigations are warranted to evaluate its mechanism and probable clinical use.

### Summary Of The Invention

This invention takes advantage of the synergistic properties of taurolidine with various oncologic drugs. The present invention provides a therapeutic nanoparticle as defined in claim 1. Preferred embodiments are defined in the dependent claims.
Disclosed herein are nanoparticles comprising one or more oncologic drugs and taurolidine, with or without additional excipients (e.g., a buffer so as to provide enhanced hydrolytic stability of the taurolidine and/or the one or more oncologic drugs and the taurolidine), whereby to provide the simultaneous delivery of the one or more oncologic drugs and taurolidine, thereby harnessing the synergistic effect of taurolidine on these oncologic drugs.

Further disclosed are nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating which is configured to release the one or more oncologic drugs and taurolidine locally to the site of a cancer, e.g., a tumor. In one preferred form of the invention, the coating is configured to prevent premature exposure of the one or more oncologic drugs and taurolidine to the body prior to delivery to the site of the cancer, e.g., a tumor. This can be important in order to prevent undesirable side effects from the one or more oncologic drugs, the premature hydrolization of the taurolidine, etc. According to the invention, the coating comprises an absorbable polymer.

Disclosed herein are nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating, wherein the coating is configured to target the nanoparticle to the site of a cancer (e.g., a tumor) so as to improve the efficacy of the one or more oncologic drugs and taurolidine for treatment of the cancer. In one preferred form of the invention, the coating comprises binding molecules which are configured to target delivery of the nanoparticle to specific tissue.

And in one preferred form of the invention, the nanoparticles are specifically configured for the treatment of neuroblastoma and/or other specific cancers.

There is further disclosed a therapeutic nanoparticle comprising:
at least one oncologic drug; and
taurolidine,
whereby to provide the simultaneous delivery of the at least one oncologic drug and taurolidine, thereby harnessing the synergistic effect of taurolidine on the at least one oncologic drug.

In another preferred form of the present invention, there is provided a therapeutic nanoparticle according to the present invention for use in a method for treating cancer, the method comprising:
providing said therapeutic nanoparticle and
delivering said therapeutic nanoparticle to a body so as to provide the simultaneous delivery of the at least one oncologic drug and taurolidine, thereby harnessing the synergistic effect of taurolidine on the at least one oncologic drug.

### Detailed Description Of The Preferred Embodiments

This invention takes advantage of the synergistic properties of taurolidine with various oncologic drugs. More particularly, this disclosure comprises the provision and use of nanoparticles comprising one or more oncologic drugs and taurolidine, with or without additional excipients (e.g., a buffer so as to provide enhanced hydrolytic stability of the taurolidine and/or the one or more oncologic drugs and the taurolidine), whereby to provide the simultaneous delivery of the one or more oncologic drugs and taurolidine, thereby harnessing the synergistic effect of taurolidine on these oncologic drugs.

Disclosed herein are nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating which is configured to release the one or more oncologic drugs and taurolidine locally to the site of a cancer, e.g., a tumor. In one preferred form of the invention, the coating is configured to prevent premature exposure of the one or more oncologic drugs and taurolidine to the body prior to delivery to the site of the cancer, e.g., a tumor. This can be important in order to prevent undesirable side effects from the one or more oncologic drugs, the premature hydrolization of the taurolidine, etc. According to the invention, the coating comprises an absorbable polymer.

Disclosed herein are nanoparticles comprising one or more oncologic drugs and taurolidine, with or without additional excipients (e.g., a buffer so as to provide enhanced hydrolytic stability of the taurolidine and/or the one or more oncologic drugs and the taurolidine), and further comprising a coating, wherein the coating is configured to target the nanoparticle to the site of a cancer (e.g., a tumor) so as to improve the efficacy of the one or more oncologic drugs and taurolidine for treatment of the cancer. In one preferred form of the invention, the coating comprises binding molecules which are configured to target delivery of the nanoparticle to specific tissue.

And in one preferred form of the invention, the nanoparticles are specifically configured for the treatment of neuroblastoma and/or other specific cancers.

More particularly, this invention takes advantage of the synergistic properties of taurolidine with various oncologic drugs by encapsulating the taurolidine and various oncologic drugs in specific nanoparticle systems that are designed to release the oncologic drug and taurolidine locally to the site of the cancer, e.g., a tumor.

A number of absorbable polymer systems can be used to optimize the release properties of the oncological drug(s) and taurolidine, especially those created from combinations of copolymers and multimers derived from polymers structured from l-lactide, glycolide, e-caprolactone, p-dioxanone, and trimethylene carbonate. These may also be associated with glycols such as polyethylene glycols (PEGs), which can either be linear or multi-arm structures.

Optimization of the systems containing taurolidine, the oncologic drug(s) and the polymers in a nanoparticle yields an improved treatment for cancer in general, and neuroblastoma in particular.

Additionally, research has shown cannabinoid activity on neuroblastoma N-type calcium channels, glycine receptor channels and voltage gated potassium channels. Each of these is believed to be specific for neural tissue and is not believed to be present in reticuloendothelial system (RES) cells. Therefore, providing the nanoparticle with binding molecules to target neural tissue (e.g., neuroblastoma N-type calcium channels, glycine receptor channels and voltage gated potassium channels) enhances the targeted delivery of the nanoparticle to neural tissue and hence enhances the effectiveness of the oncologic drug(s) (which is further enhanced by the presence of the synergistic taurolidine).

It is generally important that the binding molecules of the nanoparticle have no other significant biologic activity. To achieve specific binding with no other significant biologic activity, a fragment antigen-binding (Fab) fragment of a monoclonal antibody (which is a region on an antibody that binds to antigens) is utilized. However, since there is a recently described syndrome of severe autoimmune encephalitis resulting from anti-voltage gated potassium channel antibodies, anti-voltage gated potassium channel antibodies (e.g., KvR) are preferably not used as a target. Other targets are also reporting cases of autoimmune encephalitis as the etiology of paraneoplastic syndromes. So far none of these targets are as severe as KvR disease, but this may be due to random chance. Therefore, care must be taken in selecting the binding molecules used to target neural tissue.

In one preferred form of the present invention, the coating for the nanoparticle comprises a monoclonal antibody against N-type calcium channels (e.g., an anti-N-type calcium channel exofacial Fab fragment) for causing the nanoparticle to bind to neural tissue (e.g., to a neuroblastoma tumor), such that the one or more oncologic drugs and the taurolidine are simultaneously delivered (via the targeted nanoparticle) to the neural tissue, with the taurolidine providing a synergistic effect for the one or more oncologic drugs, whereby to provide enhanced efficacy for the one or more oncologic drugs against the targeted neural tissue.

In one particularly preferred form of the present invention, the anti-N-type calcium channel exofacial Fab fragment incorporated in the coating for the nanoparticle comprises Caᵥ2.2, or a binding equivalent thereof.

There is disclosed a nanoparticle containing cytotoxic chemotherapeutic drug(s) and synergistic taurolidine in an appropriate buffer in order to provide enhanced hydrolytic stability of the taurolidine and/or the one or more oncologic drugs and the taurolidine. The surface of the nanoparticle is a lipid envelope or polymer which regulates the release properties of the chemotherapeutic drug(s) and synergistic taurolidine. The surface of the nanoparticle preferably includes binding molecules to target neural tissue.

There is disclosed a nanoparticle which may be used to treat neuroblastoma in a patient, wherein the nanoparticle comprises a chemotherapeutic drug(s) and a synergistic quantity of taurolidine, with the chemotherapeutic drug(s) and taurolidine being encapsulated in a polymer which regulates the release properties of the chemotherapeutic drug and taurolidine.

### References

1. Brodeur, G.; Seeger, R.; Schwab, M; Varmus, H.; Bishop, J. (1984). "Amplification of N-myc in untreated human neuroblastomas correlates with advanced disease stage". Science. 224 (4653): 1121-4.
2. Wang, Kai; Diskin, Sharon J.; Zhang, Haitao; Attiyeh, Edward F.; Winter, Cynthia; Hou, Cuiping; Schnepp, Robert W.; Diamond, Maura; Bosse, Kristopher; Mayes, Patrick A.; Glessner, Joseph; Kim, Cecilia; Frackelton, Edward; Garris, Maria; Wang, Qun; Glaberson, Wendy; Chiavacci, Rosetta; Nguyen, Le; Jagannathan, Jayanti; Saeki, Norihisa; Sasaki, Hiroki; Grant, Struan F. A.; Iolascon, Achille; Mosse, Yael P.; Cole, Kristina A.; Li, Hongzhe; Devoto, Marcella; McGrady, Patrick W.; London, Wendy B.; Capasso, Mario; Rahman, Nazneen; Hakonarson, Hakon; Maris, John M. (2011). "Integrative genomics identifies LMO1 as a neuroblastoma oncogene". Nature. 469 (7329): 216-20.
3. Diskin, Sharon J.; Hou, Cuiping; Glessner, Joseph T.; Attiyeh, Edward F.; Laudenslager, Marci; Bosse, Kristopher; Cole, Kristina; Mossé, Yaël P.; Wood, Andrew; Lynch, Jill E.; Pecor, Katlyn; Diamond, Maura; Winter, Cynthia; Wang, Kai; Kim, Cecilia; Geiger, Elizabeth A.; McGrady, Patrick W.; Blakemore, Alexandra I. F.; London, Wendy B.; Shaikh, Tamim H.; Bradfield, Jonathan; Grant, Struan F. A.; Li, Hongzhe; Devoto, Marcella; Rappaport, Eric R.; Hakonarson, Hakon; Maris, John M. (2009). "Copy number variation at 1q21.1 associated with neuroblastoma". Nature. 459 (7249): 987-91.
4. Olshan, Andrew F; Bunin, Greta R. (2000). "Epidemiology of Neuroblastoma". In Brodeur, Garrett M.; Sawada, Tadashi; Tsuchida, Yoshiaki; et al. Neuroblastoma. Amsterdam: Elsevier. pp. 33-9.
5. Menegaux, Florence; Olshan, Andrew F.; Neglia, Joseph P.; Pollock, Brad H.; Bondy, Melissa L. (2004). "Day care, childhood infections, and risk of neuroblastoma". American Journal of Epidemiology. 159 (9): 843-51.
6. Oishan, A et al "Hormone and Fertility Drug Use and the Risk of Neuroblastoma: A report from the Children's Cancer Group and Pediatric Oncology Group" American Journal of Epidemiology. 150 (9): 930-8.
7. McCall, Erin E.; Olshan, Andrew F.; Daniels, Julie L. (2005). "Maternal hair dye use and risk of neuroblastoma in offspring". Cancer Causes & Control. 16 (6): 743-8.
8. Strenger, Volker; Kerbl, Reinhold; Dornbusch, Hans Jürgen; Ladenstein, Ruth; Ambros, Peter F.; Ambros, Inge M.; Urban, Christian (2007). "Diagnostic and prognostic impact of urinary catecholamines in neuroblastoma patients". Pediatric Blood & Cancer. 48 (5): 504-9.
9. Gisselsson, David; Lundberg, Gisela; Øra, Ingrid; Höglund, Mattias (2007). "Distinct evolutionary mechanisms for genomic imbalances in high-risk and low-risk neuroblastomas". Journal of Carcinogenesis. 6: 15.
10. Yu, A. L.; Gilman, A. L.; Ozkaynak, M. F.; London, W. B.; Kreissman, S.; Chen, H. X.; Matthay, K. K.; Cohn, S. L.; Maris, J. M.; Sondel, P. (2009). "A phase III randomized trial of the chimeric anti-GD2 antibody chl4.18 with GM-CSF and IL2 as immunotherapy following dose intensive chemotherapy for high-risk neuroblastoma: Childrens Oncology Group (COG) study ANBL0032".
11. Baker, D. L.; Schmidt, M.; Cohn, S.; London, W. B.; Buxten, A.; Sandler, A.; Shimada, H.; Matthay, K. (2007). "A phase III trial of biologically-based therapy reduction for intermediate risk neuroblastoma". Journal of Clinical Oncology. 25 (18 Suppl): 9504.
12. Baker, David L.; Schmidt, Mary L.; Cohn, Susan L.; Maris, John M.; London, Wendy B.; Buxton, Allen; Stram, Daniel; Castleberry, Robert P.; Shimada, Hiroyuki; Sandler, Anthony; Shamberger, Robert C.; Look, A. Thomas; Reynolds, C. Patrick; Seeger, Robert C.; Matthay, Katherine K. (2010). "Outcome after Reduced Chemotherapy for Intermediate-Risk Neuroblastoma". New England Journal of Medicine. 363 (14): 1313-23.
13. Pritchard, Jon; Cotterill, Simon J.; Germond, Shirley M.; Imeson, John; de Kraker, Jan; Jones, David R. (2005). "High dose melphalan in the treatment of advanced neuroblastoma: Results of a randomised trial (ENSG-1) by the European Neuroblastoma Study Group". Pediatric Blood & Cancer. 44 (4): 348-57.
14. Pearson, Andrew DJ; Pinkerton, C Ross; Lewis, Ian J; Imeson, John; Ellershaw, Caroline; Machin, David (2008). "High-dose rapid and standard induction chemotherapy for patients aged over 1 year with stage 4 neuroblastoma: a randomised trial". The Lancet Oncology. 9 (3): 247-56.
15. Matthay, K. K.; Reynolds, C. P.; Seeger, R. C.; Shimada, H.; Adkins, E. S.; Haas-Kogan, D.; Gerbing, R. B.; London, W. B.; Villablanca, J. G. (2009). "Long-Term Results for Children With High-Risk Neuroblastoma Treated on a Randomized Trial of Myeloablative Therapy Followed by 13-cis-Retinoic Acid: A Children's Oncology Group Study". Journal of Clinical Oncology. 27 (7): 1007-13.
16. Berthold, Frank; Boos, Joachim; Burdach, Stefan; Erttmann, Rudolf; Henze, Günter; Hermann, Johann; Klingebiel, Thomas; Kremens, Bernhard; Schilling, Freimut H; Schrappe, Martin; Simon, Thorsten; Hero, Barbara (2005). "Myeloablative megatherapy with autologous stem-cell rescue versus oral maintenance chemotherapy as consolidation treatment in patients with high-risk neuroblastoma: a randomised controlled trial". The Lancet Oncology. 6 (9): 649-58.
17. Kreissman, S. G.; Villablanca, J. G.; Diller, L.; London, W. B.; Maris, J. M.; Park, J. R.; Reynolds, C. P.; von Allmen, D.; Cohn, S. L.; Matthay, K. K. (2007). "Response and toxicity to a dose-intensive multi-agent chemotherapy induction regimen for high risk neuroblastoma (HR-NB): A Children's Oncology Group (COG A3973) study". Journal of Clinical Oncology. 25 (18 Suppl): 9505.
18. Ladenstein, R.; Valteau-Couanet, D.; Brock, P.; Yaniv, I.; Castel, V.; Laureys, G.; Malis, J.; Papadakis, V.; Lacerda, A.; Ruud, E.; Kogner, P.; Garami, M.; Balwierz, W.; Schroeder, H.; Beck-Popovic, M.; Schreier, G.; Machin, D.; Potschger, U.; Pearson, A. (2010). "Randomized Trial of Prophylactic Granulocyte Colony-Stimulating Factor During Rapid COJEC Induction in Pediatric Patients With High-Risk Neuroblastoma: The European HR-NBL1/SIOPEN Study". Journal of Clinical Oncology. 28 (21): 3516-24.
19. Clinical trial number NCT00410631 for "Observation, Combination Chemotherapy, Radiation Therapy, and/or Autologous Stem Cell Transplant in Treating Young Patients With Neuroblastoma" at ClinicalTrials.gov.
20. George, Rani E.; Li, Shuli; Medeiros-Nancarrow, Cheryl; Neuberg, Donna; Marcus, Karen; Shamberger, Robert C.; Pulsipher, Michael; Grupp, Stephan A.; Diller, Lisa (2006). "High-Risk Neuroblastoma Treated With Tandem Autologous Peripheral-Blood Stem Cell-Supported Transplantation: Long-Term Survival Update". Journal of Clinical Oncology. 24 (18): 2891-6.
21. Clinical trial number NCT01175356 for "Induction Therapy Including 131 I-MIBG and Chemotherapy in Treating Patients With Newly Diagnosed High-Risk Neuroblastoma Undergoing Stem Cell Transplant, Radiation Therapy, and Maintenance Therapy With Isotretinoin" at ClinicalTrials.gov.
22. Kushner, B. H.; Kramer, K.; Modak, S.; Cheung, N.-K. V. (2006). "Irinotecan Plus Temozolomide for Relapsed or Refractory Neuroblastoma". Journal of Clinical Oncology. 24 (33): 5271-6.
23. "NANT Home Page".
24. Karlisch C, Harati K, Chromik AM, Bulut D, Klein- Hitpass L, Goertz O, Hirsch T, Lehnhardt M, Uhl W and Daigeler A. Effects of TRAIL and taurolidine on apoptosis and proliferation in human rhabdomyosarcoma, leiomyosarcoma and epithelioid cell sarcoma. International journal of oncology. 2013; 42(3):945-956.
25. Harati K et al, TRAIL and Taurolidine Enhance the Anticancer Activity of Doxorubicin, Trabectedin and Mafosamide in HT1080 Human Fribrosarcoma Cells Anticancer Research July 2012 (32) 72967-72984.
26. Martinotti S et al, In vitro screening of synergistic ascorbate-drug combinations for the treatment of malignant mesothelioma. Toxicology in Vitro (25)8, 1568-1574 (2011).
27. Daigeler A et al, Synergistic apoptic effects of taurolidine and TRAIL on squamous carcinoma cells of the esophagus. Intl J of Oncology (32) 1205-1220 (2008).
28. Chromik et al, Synergistic effects in apoptosis induction by taurolidine and TRAIL in HCT-15 colon carcinoma cells. Journal of Investigative Surgery (20) 339-48 (2007).
29. Braumann C et al, Local and systemic chemotherapy with taurolidine and taurolidine/heparin in colon cancer-bearing rats undergoing laparotomy. Clinical and Experimental Metastasis (20) 387-394 (2003).
30. Stendel R, et al, Enhancement of Fas-ligand-mediated programmed cell death by taurolidine. Anticancer Research (23) 2309-2314.
31. Braumann C et al, Influence of intraperitoneal and systemic application of taurolidine/heparin during laparoscopy on intraperitoneal and subcutaneous tumor growth in rats. Clinical and Experimental Metastasis (18) 547-552.
32. Monson J et al, Taurolidine inhibits tumor necrosis factor (TNF) toxicity-new evidence of TNF and endotoxin synergy. J of the European Surgical Oncology and the British Association of Surgical Oncology (19)226-231 (1993).
33. Eschenburg, et al Taurolidine cooperates with antineoplastic drugs in neeuroblatoma cells Genes Cancer (5) 460-469 (2014).

## Claims

1. A therapeutic nanoparticle comprising:
at least one oncologic drug; and
taurolidine,
whereby to provide the simultaneous delivery of the at least one oncologic drug and taurolidine, thereby harnessing the synergistic effect of taurolidine on the at least one oncologic drug;
and further comprising a coating, which is configured to release the at least one oncologic drug and taurolidine locally to the site of a cancer, wherein the coating comprises an absorbable polymer and wherein the coating is created from combinations of copolymers and multimers derived from polymers structured from at least one from the group consisting of I-lactide, glycolide, e-caprolactone, p-dioxanone, and trimethylene carbonate.

2. A therapeutic nanoparticle according to claim 1, wherein the at least one oncologic drug comprises at least one from the group consisting of tumor necrosis factor (TNF), an antineoplastic drug, a cytotoxic drug, vincristine and doxorubicin.

3. A therapeutic nanoparticle according to claim 1, wherein the therapeutic nanoparticle further comprises at least one excipient.

4. A therapeutic nanoparticle according to claim 3, wherein the at least one excipient comprises a buffer so as to provide enhanced hydrolytic stability of the taurolidine and/or the at least one oncologic drug and the taurolidine.

5. A therapeutic nanoparticle according to claim 1, wherein the coating further comprises glycols.

6. A therapeutic nanoparticle according to claim 1, wherein the coating comprises binding molecules which are configured to target delivery of the nanoparticle to specific tissue.

7. A therapeutic nanoparticle according to claim 6, wherein the binding molecules comprise a fragment antigen-binding (Fab) fragment of a monoclonal antibody.

8. A therapeutic nanoparticle according to claim 6, wherein the binding molecules are configured to target neural tissue.

9. A therapeutic nanoparticle according to claim 8, wherein the binding molecules are configured to target at least one from the group consisting of neuroblastoma N-type calcium channels, glycine receptor channels and voltage gated potassium channels.

10. A therapeutic nanoparticle according to claim 6, wherein the binding molecules are embedded in or covalently bound to the surface of the nanoparticle.

11. A therapeutic nanoparticle according to any one of the preceding claims for use in a method of treating cancer, the method comprising
providing said therapeutic nanoparticle and
delivering said therapeutic nanoparticle to a body so as to provide the simultaneuous delivery of the at least one oncologic drug and tarurolidine.

## Patentansprüche

1. Therapeutisches Nanopartikel, umfassend:
mindestens ein Krebsarzneimittel; und
Taurolidin,
um die gleichzeitige Verabreichung des mindestens einen Krebsarzneimittels und von Taurolidin bereitzustellen, wodurch die synergistische Wirkung von Taurolidin auf das mindestens eine Krebsarzneimittel nutzbar gemacht wird;
und weiter umfassend eine Beschichtung, die konfiguriert ist, um das mindestens eine Krebsarzneimittel und Taurolidin lokal an der Stelle eines Krebses freizusetzen, wobei die Beschichtung ein absorbierbares Polymer umfasst und wobei die Beschichtung aus Kombinationen von Copolymeren und Multimeren erzeugt wird, die aus Polymeren abgeleitet sind, die aus mindestens einem aus der Gruppe, bestehend aus L-Lactid, Glycolid, e-Caprolacton, p-Dioxanon und Trimethylencarbonat, strukturiert sind.

2. Therapeutisches Nanopartikel nach Anspruch 1, wobei das mindestens eine Krebsarzneimittel mindestens eines aus der Gruppe umfasst, die aus Tumornekrosefaktor (TNF), einem antineoplastischen Arzneimittel, einem cytotoxischen Arzneimittel, Vincristin und Doxorubicin besteht.

3. Therapeutisches Nanopartikel nach Anspruch 1, wobei das therapeutische Nanopartikel weiter mindestens einen Hilfsstoff umfasst.

4. Therapeutisches Nanopartikel nach Anspruch 3, wobei der mindestens eine Hilfsstoff einen Puffer umfasst, um somit verbesserte hydrolytische Stabilität des Taurolidin und/oder des mindestens einen Krebsarzneimittels und des Taurolidin bereitzustellen.

5. Therapeutisches Nanopartikel nach Anspruch 1, wobei die Beschichtung weiter Glycole umfasst.

6. Therapeutisches Nanopartikel nach Anspruch 1, wobei die Beschichtung Bindemoleküle umfasst, die konfiguriert sind, um die Bereitstellung des Nanopartikels an spezifisches Gewebe zu richten.

7. Therapeutisches Nanopartikel nach Anspruch 6, wobei die Bindemoleküle ein Fragment eines Antigen-bindenden (Fab) Fragments eines monoklonalen Antikörpers umfasst.

8. Therapeutisches Nanopartikel nach Anspruch 6, wobei die Bindemoleküle konfiguriert sind, um an Nervengewebe anzusetzen.

9. Therapeutisches Nanopartikel nach Anspruch 8, wobei die Bindemoleküle konfiguriert sind, um mindestens bei einem aus der Gruppe, bestehend aus Neuroblastom-Calciumkanälen vom N-Typ, Glycin-Rezeptorkanälen und spannungsabhängigen Kaliumkanälen, anzusetzen.

10. Therapeutisches Nanopartikel nach Anspruch 6, wobei die Bindemoleküle in der Oberfläche des Nanopartikels eingebettet sind oder an diese kovalent gebunden sind.

11. Therapeutisches Nanopartikel nach einem der vorstehenden Ansprüche zur Verwendung in einem Verfahren zur Behandlung von Krebs, wobei das Verfahren umfasst
Bereitstellen des therapeutischen Nanopartikels und
Verabreichen des therapeutischen Nanopartikels an einen Körper, um somit die gleichzeitige Verabreichung des mindestens einen Krebsarzneimittels und von Taurolidin bereitzustellen.

## Revendications

1. Nanoparticule thérapeutique comprenant :
au moins un médicament oncologique ; et
de la taurolidine,
selon laquelle il est possible de fournir l'administration simultanée du au moins un médicament oncologique et de la taurolidine, exploitant ainsi l'effet synergique de la taurolidine sur le au moins un médicament oncologique ;
et comprenant en outre une enveloppe, qui est configurée pour libérer le au moins un médicament oncologique et la taurolidine localement au site d'un cancer, dans laquelle l'enveloppe comprend un polymère absorbable et dans laquelle l'enveloppe est créée à partir d'associations de copolymères et de multimères issus de polymères structurés à partir d'au moins l'un parmi le groupe consistant en le l-lactide, le glycolide, l'e-caprolactone, la p-dioxanone, et le carbonate de triméthylène.

2. Nanoparticule thérapeutique selon la revendication 1, dans laquelle le au moins un médicament oncologique comprend au moins l'un parmi le groupe consistant en le facteur de nécrose tumorale (TNF), un médicament antinéoplasique, un médicament cytotoxique, la vincristine et la doxorubicine.

3. Nanoparticule thérapeutique selon la revendication 1, dans laquelle la nanoparticule thérapeutique comprend en outre au moins un excipient.

4. Nanoparticule thérapeutique selon la revendication 3, dans laquelle le au moins un excipient comprend un tampon afin de fournir une stabilité hydrolytique améliorée de la taurolidine et/ou du au moins un médicament oncologique et de la taurolidine.

5. Nanoparticule thérapeutique selon la revendication 1, dans laquelle l'enveloppe comprend en outre des glycols.

6. Nanoparticule thérapeutique selon la revendication 1, dans laquelle l'enveloppe comprend des molécules de liaison qui sont configurées pour cibler l'administration de la nanoparticule à un tissu spécifique.

7. Nanoparticule thérapeutique selon la revendication 6, dans laquelle les molécules de liaison comprennent un fragment type fragment de liaison à l'antigène (Fab) d'un anticorps monoclonal.

8. Nanoparticule thérapeutique selon la revendication 6, dans laquelle les molécules de liaison sont configurées pour cibler un tissu nerveux.

9. Nanoparticule thérapeutique selon la revendication 8, dans laquelle les molécules de liaison sont configurées pour cibler au moins l'un parmi le groupe consistant en des canaux calciques de type N de neuroblastome, des récepteurs-canaux de la glycine et des canaux potassiques voltage-dépendants.

10. Nanoparticule thérapeutique selon la revendication 6, dans laquelle les molécules de liaison sont incorporées dans ou liées de manière covalente à la surface de la nanoparticule.

11. Nanoparticule thérapeutique selon l'une quelconque des revendications précédentes pour utilisation dans un procédé de traitement du cancer, le procédé comprenant
la fourniture de ladite nanoparticule thérapeutique et
l'administration de ladite nanoparticule thérapeutique à un corps afin de fournir l'administration simultanée du au moins un médicament oncologique et de la taurolidine.
